(19) Europäisches Patentamt
European Patent Office
Office européen des brevets

(11) **EP 4 361 220 A1**

(12) **EUROPEAN PATENT APPLICATION**

(43) Date of publication:
**01.05.2024 Bulletin 2024/18**

(21) Application number: **23178123.8**

(22) Date of filing: **07.06.2023**

(51) International Patent Classification (IPC):
**C08L 97/02** (2006.01)   **C12N 9/24** (2006.01)
**C12P 19/02** (2006.01)   **C12P 7/06** (2006.01)

(52) Cooperative Patent Classification (CPC):
**C08L 97/02; C12N 9/2402; C12P 7/06;**
C12P 2203/00; C12Y 302/01015

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.10.2022 TW 111141185**

(71) Applicant: **Yang, Fu-Hsiang**
**Taiwan 510 (TW)**

(72) Inventor: **Yang, Fu-Hsiang**
**Taiwan 510 (TW)**

(74) Representative: **Delbarba, Andrea**
**Bugnion S.p.A.**
**Viale Lancetti, 17**
**20158 Milano (IT)**

(54) **METHOD FOR PREPARING FIBER MATERIAL FROM BANANA PSEUDOSTEM**

(57)    A method for preparing a fiber material from a banana pseudostem, which includes subjecting the banana pseudostem to a pressing treatment to obtain a pressed banana pseudostem, subjecting the pressed banana pseudostem to a fermentation reaction with *Saccharomyces cerevisiae* to obtain a fermented culture, subjecting the fermented culture to a simultaneous saccharification and fermentation process with pectinase to obtain a fermented product, subjecting the fermented product to a solid-liquid separation treatment to obtain a solid fraction, and subjecting the solid fraction to an alkali treatment, a water washing treatment, a bleaching treatment, a softening treatment, and a drying treatment in sequence, so as to obtain the fiber material.

40-60 dtex      0.9-5.0 dtex

FIG.4

EP 4 361 220 A1

**Description**

The present disclosure relates to a method for preparing a fiber material from a banana pseudostem.

The banana plant (scientific name: *Musa* × *paradisiaca*) is a herbaceous perennial of the family Musaceae, and the fruit of the banana plant is the fourth most important fruit crop in the world. After the banana fruit is harvested, the remaining banana pseudostem, which is discarded, will cause pest (such as ants and weevils) infestation, and the emission of greenhouse gases (such as methane) will occur during the decay of banana pseudostem. Therefore, those skilled in the art still strive to develop an effective way to reuse banana pseudostem waste, thereby reducing pest infestation and environmental pollution.

The cellulose, hemicellulose, and lignin contained in the banana pseudostem are intertwined and wrapped to form a complex and tough network structure. Therefore, the banana pseudostem must be subjected to a process for delignification before recycling. Methods commonly used for delignification include physical methods (such as scraping methods) and chemical methods (such as acid treatments and alkali treatments). However, these methods might cause problems such as poor fiber extraction, loss of fine fibers (such as cotton-like fibers), and low yields.

Recently, microorganisms and enzymes have been applied in the reprocessing of agricultural waste and the reuse of fibers. For instance, CN 111996603 A discloses a biological extraction method of pineapple leaf fiber, which includes subjecting a pineapple leaf fiber raw material to an enzyme treatment, an enzyme deactivation and rinsing treatment, an ultrasonic treatment, and a drying treatment in sequence. The enzyme used in the enzyme treatment may be selected from the group consisting of laccase, xylanase, and pectinase. Before the enzyme treatment, the pineapple leaf fiber raw material can be subjected to a microbial treatment using *Aspergillus niger* spore. In the examples of CN 111996603 A, the pineapple leaf fiber raw material was treated with various enzyme solutions, and then the resultant pineapple leaf fibers were analyzed for residual sericin, breaking strength, thickness, softness, and color. The results show that, in comparison with the use of pectinase alone, high quality pineapple leaf fiber can be obtained by using a combination of laccase and xylanase to treat the pineapple leaf fiber raw material, indicating that not all enzymes with delignification effect are suitable for extraction of pineapple leaf fiber.

In spite of the aforesaid, there is still a need to develop a method for preparing a fiber material from a banana pseudostem, which can effectively reduce banana pseudostem waste and increase fiber yield.

Therefore, an object of the disclosure is to provide a method for preparing a fiber material from a banana pseudostem, which can alleviate at least one of the drawbacks of the prior art.

According to an aspect of the disclosure, there is provided a method for preparing a fiber material from a banana pseudostem according to claim 1.

According to an aspect of the disclosure, there is provided a fiber material according to claim 5.

Other features and advantages of the disclosure will become apparent in the following detailed description of the embodiment(s) with reference to the accompanying drawings. It is noted that various features may not be drawn to scale.

Figure 1 is a photograph showing the segmented banana pseudostems prepared in Example 1, *infra.*
Figure 2 is a photograph showing the pressed banana pseudostems prepared in Example 1, *infra.*
Figure 3 is a photograph showing the fermented product prepared in Example 1, *infra.*
Figure 4 is a photograph showing the fiber material prepared in Example 1, *infra.*

For the purpose of this specification, it will be clearly understood that the word "comprising" means "including but not limited to", and that the word "comprises" has a corresponding meaning.

It is to be understood that, if any prior art publication is referred to herein, such reference does not constitute an admission that the publication forms a part of the common general knowledge in the art, in Taiwan or any other country.

Unless defined otherwise, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

The present disclosure provides a method for preparing a fiber material from a banana pseudostem, which includes:

(a) subjecting the banana pseudostem to a pressing treatment, so as to obtain a pressed banana pseudostem;
(b) subjecting the pressed banana pseudostem to a fermentation reaction with *Saccharomyces cerevisiae,* so as to obtain a fermented culture;
(c) subjecting the fermented culture to a simultaneous saccharification and fermentation process with pectinase, so as to obtain a fermented product;
(d) subjecting the fermented product to a solid-liquid separation treatment, so as to obtain a solid fraction; and

(e) subjecting the solid fraction to an alkali treatment, a water washing treatment, a bleaching treatment, a softening treatment, and a drying treatment in sequence, so as to obtain the fiber material.

In certain embodiments, the banana pseudostem may be obtained from a banana plant selected from the group consisting of *Cavendish* spp., *Musa* spp., *Ensete* spp., and combinations thereof. In an exemplary embodiment, the banana pseudostem is obtained from a banana plant of *Cavendish* spp.

As used herein, the terms "pseudostem" and "stubble" can be used interchangeably.

According to the present disclosure, the pressing treatment, the fermentation reaction, the simultaneous saccharification and fermentation process, the solid-liquid separation treatment, the alkali treatment, the water washing treatment, the bleaching treatment, the softening treatment, and the drying treatment may be performed using techniques well-known to those skilled in the art. It should be noted that the procedures and operating conditions for the aforesaid treatments may be adjusted according to practical requirements and factors (such as the water content and amount of the banana pseudostem).

In certain embodiments, in step (a), the pressing treatment is conducted using an oil pressure type presser, so that approximately 30% to 50% of the juice of the banana pseudostem can be removed.

In certain embodiments, before performing step (a), the banana pseudostem may be subjected to a segmentation treatment. In an exemplary embodiment, the banana pseudostem is segmented to have a length ranging from 60 cm to 100 cm.

As used herein, the term "fermentation" can be used interchangeably with the terms "culturing" and "cultivation".

It should be noted that the procedures and operating conditions for the fermentation reaction may be adjusted according to practical requirements and factors (such as the content ratio of *Saccharomyces cerevisiae* to the pressed banana pseudostem), so as to achieve an optimum fermentation effect.

In certain embodiments, in step (b), the fermentation reaction may be conducted at a temperature ranging from 20°C to 32°C. In an exemplary embodiment, the fermentation reaction is conducted at 28°C.

In certain embodiments, in step (b), during the fermentation reaction, a sugar may be added to adjust the sugar content. In an exemplary embodiment, the sugar content is maintained at 18 degrees Brix to 20 degrees Brix.

In certain embodiments, the sugar may be selected from the group consisting of glucose, fructose, sucrose, table sugar mainly composed of sucrose, and combinations thereof.

Examples of the table sugar mainly composed of sucrose may include, but are not limited to, rock sugar, white sugar, soft white sugar, and brown sugar. In certain embodiments, the table sugar mainly composed of sucrose is white sugar.

In certain embodiments, the fermented culture obtained in step (b) may be further subjected to a distillation treatment, so as to obtain bioethanol and a distillation residue which can be used as an organic fertilizer. In an exemplary embodiment, the distillation treatment is conducted at a temperature ranging from 78°C to 100°C.

In certain embodiments, in step (c), the simultaneous saccharification and fermentation process may be conducted at a temperature ranging from 15°C to 55°C. In an exemplary embodiment, the simultaneous saccharification and fermentation process is conducted at 50°C.

In certain embodiments, in step (c), the pectinase suitable for use in this disclosure may be obtained as commercial products. In an exemplary embodiment, the pectinase is a pectic enzyme (Manufacturer: North Mountain Supply) purchased from Bake n Brew Homebrew Supply.

In certain embodiments, in step (e), the alkali treatment may be conducted using an alkaline solution at a temperature ranging from 60°C to 90°C for 3 hours.

In certain embodiments, the alkaline solution may be selected from the group consisting of a potassium hydroxide solution, a sodium hydroxide solution, a calcium carbonate solution, and combinations thereof. In an exemplary embodiment, the alkaline solution is a potassium hydroxide solution having a concentration ranging from 2 wt% to 4 wt%. In another exemplary embodiment, the alkaline solution is a 2 wt% potassium hydroxide solution.

In certain embodiments, in step (e), the water washing treatment may be conducted by washing the solid fraction with a high-pressure water column having a pressure of 100 bar, so as to remove lignin.

In certain embodiments, in step (e), the bleaching treatment may be conducted using a bleaching agent at a temperature ranging from 20°C to 150°C for 3 hours.

In certain embodiments, the bleaching agent may be selected from the group consisting of a hydrogen peroxide solution, a sodium hypochlorite solution, a calcium hypochlorite solution, a sodium perborate solution, and combinations thereof. In an exemplary embodiment, the bleaching agent is a hydrogen peroxide solution having a concentration ranging from 2 wt% to 4 wt%. In another exemplary embodiment, the bleaching agent is a 2 wt% hydrogen peroxide solution.

In certain embodiments, in step (e), the softening treatment may be conducted using an anionic oil for 90 minutes. In an exemplary embodiment, the anionic oil is a phosphorylated synthetic fat-liquoring agent (Product name: phosphorylated synthetic oil; Trade name: SOFCON SWK) purchased from Giant Sun Fat & Oil Chemical Co., Ltd.

In certain embodiments, in step (e), the drying treatment may be conducted at a temperature ranging from 40°C to 100°C for a time period ranging from 90 minutes to 120 minutes. In an exemplary embodiment, the drying treatment is conducted at 100°C for 120 minutes.

In certain embodiments, the fiber material obtained in step (e) may be further subjected to a bale opening treatment, so as to obtain two fibers having tow finenesses of about 0.9-5.0 decitex (dtex) and 40-60 dtex, respectively. In an exemplary embodiment, the fiber material contains 80 wt% of the fiber having a tow fineness of about 0.9-5.0 dtex and 20 wt% of the fiber having a tow fineness of about 40-60 dtex.

The present disclosure also provides a fiber material, which is prepared by a method described above.

According to the present disclosure, the fiber material may be further subjected to a processing treatment, so as to obtain a textile product. Examples of the textile product may include, but are not limited to, yarns, cotton yarns, and plaits (such as clothes, canvas, cord fabrics, nonwovens, and shoelaces).

According to the present disclosure, the fiber material may be manufactured into a fiber product using techniques well-known to those skilled in the paper industry or the artificial fiber industry. Examples of the fiber product may include, but are not limited to, papers, furniture, tableware, shoes, leathers, and filter media.

The disclosure will be further described by way of the following examples. However, it should be understood that the following examples are solely intended for the purpose of illustration and should not be construed as limiting the disclosure in practice.

## EXAMPLES

### General Experimental Materials:

1. Sources of banana pseudostem

The banana pseudostems used in the following experiments were collected from Nantou County, Jiji Township, Taiwan; Yunlin County, Citong Township, Taiwan; Kaohsiung City, Dashu District, Taiwan; and Pingtung County, Changzhi Township, Taiwan.

2. *Saccharomyces cerevisiae* powder and pectinase

*Saccharomyces cerevisiae* powder (Product name: Fermentis SafAle™ US-05 ale yeast; Manufacturer: Lesaffre Yeast Corporation) and pectinase (Product name: Pectic Enzyme; Manufacturer: North Mountain Supply) used in the following experiments were purchased from Bake n Brew Homebrew Supply.

### Example 1. Preparation of fiber material of the present disclosure

A. Preparation of *Saccharomyces cerevisiae* inoculum

0.122 kg of *Saccharomyces cerevisiae* powder was evenly mixed with 6 kg of granulated sugar (purchased from Taiwan Sugar Corporation) and 18 L of reverse osmosis water, so as to obtain a *Saccharomyces cerevisiae* inoculum. B. Preparation of fiber material

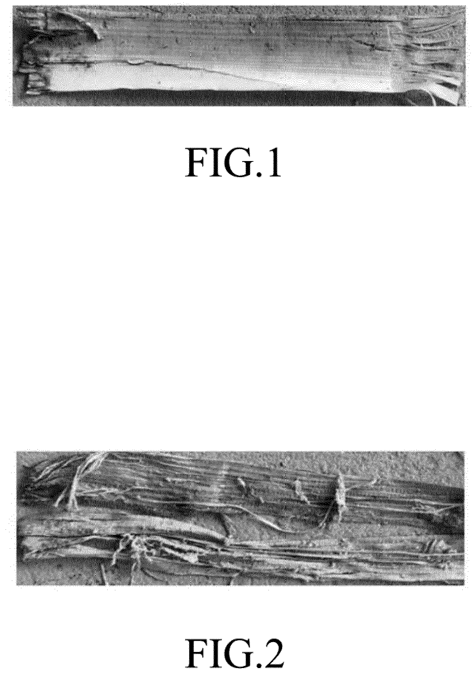

First, 30 kg of the banana pseudostems described in section 1 of "General Experimental Materials" were cut into segments, each having a length ranging from 60 cm to 100 cm (as shown in Figure 1), by using a cutting machine. Next, the segmented banana pseudostems were subjected to a pressing treatment, so as to obtain pressed banana pseudostems (as shown in Figure 2).

The pressed banana pseudostems were placed in a fermenter, and then the *Saccharomyces cerevisiae* inoculum prepared in section A of this example was inoculated in an amount of 0.5% (v/v) into the fermenter, followed by conducting a fermentation reaction at 28°C for 120 hours, so as to obtain a fermented culture. During the fermentation reaction, the sugar content (degrees Brix) was measured using a saccharometer, and the sugar content was maintained at 18 degrees Brix to 20 degrees Brix by adding granulated sugar.

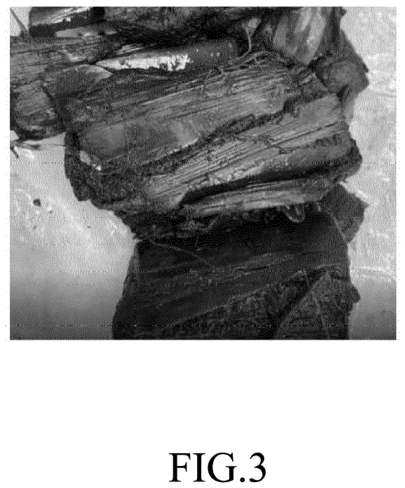

Thereafter, 0.027 mL of a 0.005 wt% pectinase solution was added to the fermented culture and mixed evenly, followed by conducting a simultaneous saccharification and fermentation process at 50°C for 120 hours, so as to obtain a fermented product (as shown in Figure 3). The fermented product was then subjected to a solid-liquid separation treatment using a filter for 2 hours, so as to obtain a solid fraction.

Afterward, the solid fraction was subjected to an alkali treatment by immersing the solid fraction in a 2 wt% potassium hydroxide solution at 90°C for 3 hours, followed by conducting a water washing treatment with a high-pressure water column having a pressure of 100 bar, so as to remove lignin.

Next, the resultant washed solid substance was subjected to a bleaching treatment by immersing the washed solid substance in a 2 wt% hydrogen peroxide solution at 100°C for 3 hours. The resultant bleached solid substance was subjected to a softening treatment by immersing the bleached solid substance in an appropriate amount of a phosphorylated synthetic fat-liquoring agent (Product name: phosphorylated synthetic oil; Trade name: SOFCON SWK; Manufacturer: Giant Sun Fat & Oil Chemical Co., Ltd) for 90 minutes, followed by conducting a drying treatment, which was performed at 100°C for 120 minutes using a hot air circulator, so as to obtain a fiber tow.

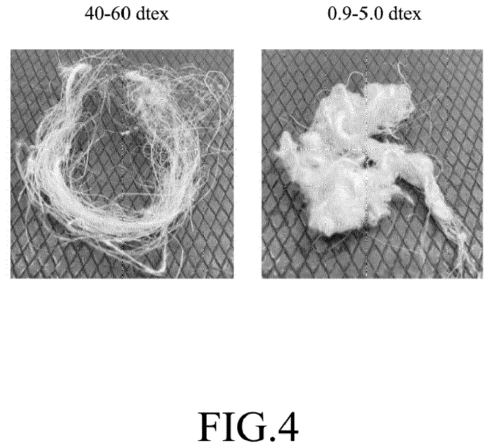

Thereafter, the fiber tow was subjected to a bale opening treatment, so as to obtain a fiber material having two different fibers with fiber fineness of about 0.9-5.0 decitex (dtex) and 40-60 dtex, respectively (as shown in Figure 4). The fiber material served as an experimental group.

In addition, the fiber material serving as a comparative group was prepared for comparison purpose using procedure that is substantially similar to that of the experimental group, except that the banana pseudostems were not subjected to a pressing treatment, and the lignin and hemicellulose of the banana pseudostems were scraped using techniques well-known to those skilled in the art.

C. Determination of fiber yield

The fiber material of each group prepared in section B of this example was subjected to weight measurement, and the fiber yield was calculated using the following Equation (I):

$$A=(B/C)\times100 \hspace{4cm} (I)$$

where A=fiber yield (%)
B=total weight of fiber material of respective group
C=total weight of banana pseudostems (i.e., 30 kg)

As shown in Table 1 below, the fiber yield determined in the experimental group was higher than that determined in the comparative group. In addition, the applicant found that the fiber material of the comparative group did not have fibers with fiber fineness of lower than 4 dtex.

These results indicate that the fiber material prepared according to the method of the present disclosure has more fiber content and fiber types, and hence can be effectively recycled.

Table 1

| Group | Fiber yield (%) |
|---|---|
| Experimental group | 8 |
| Comparative group | 2 |

**Example 2. Recycling of fermented culture**

A. Preparation of bio-alcohol and distillation residue

The fermented culture obtained in section B of Example 1 was subjected to filtration using a filter to obtain a filtrate. The filtrate was then subjected to distillation at 78°C to 100°C using a distillation machine, so as to obtain a bio-alcohol and a distillation residue.

B. Analysis of amino acid components

The distillation residue obtained in section A of this example was subjected to amino acid component analysis which was entrusted to Jackson International Standard Inspection Co., Ltd., Taiwan.

As shown in Table 2 below, the distillation residue contained various amino acids.

Table 2

| Type of amino acid | Content (mg/100 g) |
|---|---|
| Alanine | 156.02 |
| Arginine | 55.68 |
| Asparagine | 651 |

(continued)

| Type of amino acid | Content (mg/100 g) |
|---|---|
| Cystine | 480.1 |
| Glutamic acid | 247.38 |
| Glycine | 81.78 |
| Histidine | 20.15 |
| Isoleucine | 70.06 |
| Leucine | 105.01 |
| Lysine | 144.53 |
| Methionine | 8.03 |
| Phenylalanine | 60.08 |
| Proline | 55.06 |
| Threonine | 71.11 |
| Tyrosine | 41.58 |
| Valine | 87.1 |

C. Analysis of metal components

The distillation residue obtained in section A of this example was subjected to metal component analysis which was entrusted to Jackson International Standard Inspection Co., Ltd., Taiwan.

As shown in Table 3 below, the metals and the contents thereof in the distillation residue were in compliance with the regulations for organic fertilizers in the Agriculture Fertilizer Standards stipulated by the Agriculture and Food Agency, Council of Agriculture, Executive Yuan, Taiwan.

Table 3

| Type of metal | Content |
|---|---|
| Arsenic | 0.12 ppm |
| Cadmium | 0 ppm |
| Copper | 0.67 ppm |
| Lead | 0.06 ppm |
| Mercury | 0 ppm |
| Chromium | 0.06 ppm |
| Nickel | 0.14 ppm |
| Sodium | 191 ppm |
| Zinc | 12 ppm |
| Potassium peroxide | 0.81% |
| Sodium chloride | 0.035% |

Summarizing the above test results, it is clear that the method of the present disclosure can effectively prepare a fiber material from a banana pseudostem, and the fermented culture obtained during the preparation process can be further processed by distillation to obtain a bio-alcohol and a distillation residue that can be used as an organic fertilizer.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

**Claims**

1. A method for preparing a fiber material from a banana pseudostem, **characterized by** the steps of:

   (a) subjecting the banana pseudostem to a pressing treatment, so as to obtain a pressed banana pseudostem;
   (b) subjecting the pressed banana pseudostem to a fermentation reaction with *Saccharomyces cerevisiae,* so as to obtain a fermented culture;
   (c) subjecting the fermented culture to a simultaneous saccharification and fermentation process with pectinase, so as to obtain a fermented product;
   (d) subjecting the fermented product to a solid-liquid separation treatment, so as to obtain a solid fraction; and
   (e) subjecting the solid fraction to an alkali treatment, a water washing treatment, a bleaching treatment, a softening treatment, and a drying treatment in sequence, so as to obtain the fiber material.

2. The method as claimed in Claim 1, wherein in step (b), the fermentation reaction is conducted at a temperature ranging from 20°C to 32°C.

3. The method as claimed in any one of Claims 1 to 2, wherein in step (c), the simultaneous saccharification and fermentation process is conducted at a temperature ranging from 15°C to 55°C.

4. The method as claimed in any one of Claims 1 to 3, further comprising:
   subjecting the fiber material obtained in step (e) to a bale opening treatment.

5. A fiber material, which is prepared by a method according to Claim 1.

FIG.1

FIG.2

FIG.3

40-60 dtex          0.9-5.0 dtex

# FIG.4

Europäisches Patentamt
European Patent Office
Office européen des brevets

# EUROPEAN SEARCH REPORT

Application Number

EP 23 17 8123

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2012/056141 A2 (MOMPON BERNARD [FR]) 3 May 2012 (2012-05-03) * page 5, line 21 – page 6, line 17 * * page 7, line 31 – page 8, line 3 * * page 10, line 14 – line 17 * | 1-4 | INV. C08L97/02 C12N9/24 C12P19/02 C12P7/06 |
| X | BALDA SANJEEV ET AL: "Banana fibre: a natural and sustainable bioresource for eco-friendly applications", CLEAN TECHNOLOGIES AND ENVIRONMENTAL POLICY, SPRINGER BERLIN HEIDELBERG, BERLIN/HEIDELBERG, vol. 23, no. 5, 17 February 2021 (2021-02-17), pages 1389-1401, XP037481420, ISSN: 1618-954X, DOI: 10.1007/S10098-021-02041-Y [retrieved on 2021-02-17] | 5 | |
| Y | * page 1392, column 2, paragraph 3 * * page 1393, column 1, line 1 – line 19 * * page 1393, column 2, line 15 – line 30 * * page 1399, column 1, paragraph 1 * | 1-4 | |
| X | WO 2022/003634 A1 (GENCREST PRIVATE LTD [IN]) 6 January 2022 (2022-01-06) * page 1, line 37 – page 2, line 22; figure 1 * * page 3, line 26 – page 4, line 18 * | 5 | TECHNICAL FIELDS SEARCHED (IPC) C08L C12N C09J C12P |
| X | JP 4 125065 B2 (NISSHIN SPINNING) 23 July 2008 (2008-07-23) * [0020]-[0022],[0029] * | 5 | |
| X | JP 2019 194378 A (ASAHI KASEI CORP) 7 November 2019 (2019-11-07) * [0006] * | 5 | |

-/--

The present search report has been drawn up for all claims

| Place of search | Date of completion of the search | Examiner |
|---|---|---|
| Berlin | 5 February 2024 | Schönwasser, D |

EPO FORM 1503 03.82 (P04C01)

| | Europäisches Patentamt European Patent Office Office européen des brevets | **EUROPEAN SEARCH REPORT** | Application Number EP 23 17 8123 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/003635 A1 (GENCREST PRIVATE LTD [IN]) 6 January 2022 (2022-01-06) * page 11, line 25 – line 26; claims 1, 2, 10 * | 1-5 | |
| A | HARINDER SINGH OBEROI ET AL: "Ethanol production from banana peels using statistically optimized simultaneous saccharification and fermentation process", WASTE MANAGEMENT, ELSEVIER, NEW YORK, NY, US, vol. 31, no. 7, 5 February 2011 (2011-02-05), pages 1576-1584, XP028202170, ISSN: 0956-053X, DOI: 10.1016/J.WASMAN.2011.02.007 [retrieved on 2011-02-12] * the whole document * | 1-5 | |
| A | CN 108 457 113 A (GUANGXI YIKANGDA BIOTECHNOLOGY CO LTD) 28 August 2018 (2018-08-28) * claims 1,2,4 * * examples 1-4 * | 1-5 | TECHNICAL FIELDS SEARCHED (IPC) |

The present search report has been drawn up for all claims

| Place of search | Date of completion of the search | Examiner |
|---|---|---|
| Berlin | 5 February 2024 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 8123

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2024

| Patent document cited in search report | | Publication date | Patent family member(s) | | Publication date |
|---|---|---|---|---|---|
| WO 2012056141 | A2 | 03-05-2012 | AU | 2011322393 A1 | 23-05-2013 |
| | | | BR | 112013010513 A2 | 13-06-2017 |
| | | | CA | 2814816 A1 | 03-05-2012 |
| | | | CN | 103314093 A | 18-09-2013 |
| | | | CO | 6710949 A2 | 15-07-2013 |
| | | | EP | 2633017 A2 | 04-09-2013 |
| | | | ES | 2616854 T3 | 14-06-2017 |
| | | | FR | 2966700 A1 | 04-05-2012 |
| | | | IL | 225993 A | 30-11-2017 |
| | | | JP | 6325253 B2 | 16-05-2018 |
| | | | JP | 6509171 B2 | 08-05-2019 |
| | | | JP | 2013542216 A | 21-11-2013 |
| | | | JP | 2017031187 A | 09-02-2017 |
| | | | KR | 20140058396 A | 14-05-2014 |
| | | | MA | 34680 B1 | 02-11-2013 |
| | | | MX | 349768 B | 11-08-2017 |
| | | | MY | 161964 A | 15-05-2017 |
| | | | NZ | 610003 A | 25-09-2015 |
| | | | RU | 2013124366 A | 10-12-2014 |
| | | | UA | 112637 C2 | 10-10-2016 |
| | | | US | 2013280320 A1 | 24-10-2013 |
| | | | US | 2019142762 A1 | 16-05-2019 |
| | | | WO | 2012056141 A2 | 03-05-2012 |
| | | | ZA | 201303122 B | 27-08-2014 |
| WO 2022003634 | A1 | 06-01-2022 | EP | 4176109 A1 | 10-05-2023 |
| | | | JP | 2023533384 A | 02-08-2023 |
| | | | US | 2023295840 A1 | 21-09-2023 |
| | | | WO | 2022003634 A1 | 06-01-2022 |
| JP 4125065 | B2 | 23-07-2008 | CN | 1472372 A | 04-02-2004 |
| | | | HK | 1061875 A1 | 08-10-2004 |
| | | | JP | 4125065 B2 | 23-07-2008 |
| | | | JP | 2004052176 A | 19-02-2004 |
| | | | KR | 20040010138 A | 31-01-2004 |
| | | | TW | 200407470 A | 16-05-2004 |
| JP 2019194378 | A | 07-11-2019 | NONE | | |
| WO 2022003635 | A1 | 06-01-2022 | EP | 4176051 A1 | 10-05-2023 |
| | | | JP | 2023533385 A | 02-08-2023 |
| | | | US | 2023340699 A1 | 26-10-2023 |
| | | | WO | 2022003635 A1 | 06-01-2022 |
| CN 108457113 | A | 28-08-2018 | NONE | | |

EPO FORM P0459

For more details about this annex : see Official Journal of the European Patent Office, No. 12/82

**REFERENCES CITED IN THE DESCRIPTION**

**Patent documents cited in the description**

- CN 111996603 A